# EUROPEAN PATENT SPECIFICATION

(11) **EP 3 298 406 B1**
(45) Date of publication and mention of the grant of the patent: **19.08.2020**
(21) Application number: 16723775.9
(22) Date of filing: 20.05.2016
(51) Int. Cl.: G01N 33/543, G01N 33/542, G01N 33/53

(54) **A METHOD FOR DETECTING AN ANALYTE USING ELECTROMAGNETIC RADIATION**
METODE ZUR DETEKTION EINES ANALYTEN MITTELS ELEKTROMAGNETISCHER STRAHLUNG
METHODE POUR LA DETECTION D'UN ANALYTE AUX MOYEN DE RADIATION ELECTROMAGNETIQUE

(30) Priority: 22.05.2015 GB 201508832
(43) Date of publication of application: 28.03.2018
(73) Proprietor: Psyros Diagnostics Limited, Littlehampton, West Sussex BN17 5JA (GB)
(72) Inventor: ROSS, Steven, Andrew, Aldington Kent TN25 7BB (GB); RICHARDS, Julie, Faversham Kent ME13 7TE (GB); SWAYNE, Mark, Thomas, Gatton, Herne Bay Kent CT6 7QQ (GB)
(74) Representative: Elkington and Fife LLP
(86) International application number: PCT/EP2016/061435
(87) International publication number: WO 2016/188902

(56) References cited:
- WO-A1-2012/137009
- CN-A- 101 405 602
- US-A1- 2012 220 023
- US-B1- 6 743 581

## Description

The present invention relates to a method for detecting an analyte, and particularly to extending the dynamic range and/or sensitivity of an immunoassay.

Immunoassays are often used for measuring the concentration of an analyte in a sample. However, they can be limited in terms of the lower limit of detection, the upper limit of detection and the dynamic measurement range.

In a typical sandwich immunoassay, an antibody specific for an antigen of interest is attached to a polymeric support such as a sheet of polyvinylchloride or polystyrene. A drop of cell extract or a sample of serum or urine is laid on the sheet, which is washed after formation of the antibody-antigen complex. Antibody specific for a different site on the antigen is then added, and the sheet is again washed. This second antibody carries a label so that it can be detected with high sensitivity. The amount of second antibody bound to the sheet is proportional to the quantity of antigen in the sample. This assay and other variations on this type of assay are well known, see, for example, "The Immunoassay Handbook, 2nd Ed." David Wild, Ed., Nature Publishing Group, 2001.

The upper limit of detection in an immunoassay is usually limited by the total amount of reagents used. The amount of antibody that can be immobilised on a support is limited by the finite surface area of the support. When excess analyte is present in the sample, the system becomes saturated, as all of the antibody binding sites are blocked with analyte (depending upon the affinity constant of the antibody used) and unbound analyte is washed away. Therefore, the system is unable to discriminate between concentrations of analyte above saturation, typically at concentrations between 1 µM and 100 mM. In lab analysers, this problem is addressed by diluting the sample and re-running the assay. However, sample dilution is impractical for point-of-care assays.

In a homogenous sandwich assay, the first antibody, the analyte and the second antibody are all incubated simultaneously and there are no wash steps. As the analyte concentration increases, the detected signal increases, until saturation is reached. At concentrations of analyte above saturation, the analyte binds separately to the first and second antibodies. Consequently, the amount of second antibody bound to the sheet is now no longer proportional to the quantity of antigen in the sample and the detected signal decreases - an effect known as "high-dose hook". This effect can reduce the dynamic range of the assay.

US 6,743,581 relates to an integrated biosensor system for the simultaneous detection of a plurality of different types of targets.

WO 2007/107716 relates to a method for detecting an analyte in a sample of whole blood.

Therefore, there exists a need for the provision of a solution to the problem of assay saturation and/or high-dose hook, in order to extend the dynamic range and/or sensitivity of a sandwich assay.

Accordingly, the present invention provides a method for detecting an analyte in a sample comprising:
(i) providing a labelled reagent, the labelled reagent having a binding site which is capable of binding the analyte or an analogue of the analyte and a label, wherein the label is capable of absorbing the electromagnetic radiation to generate energy by non-radiative decay;
(ii) providing a device having
   a radiation source adapted to generate a series of pulses of electromagnetic radiation,
   a transducer having a pyroelectric or piezoelectric element and electrodes, which is capable of transducing energy generated by non-radiative decay into an electrical signal,
   a detector which is capable of detecting the electrical signal,
   a controller for controlling the source of electromagnetic radiation and the detector,
   wherein the device has a first and second chamber,
   the first chamber containing a first reagent proximal to the transducer, wherein the first reagent is capable of binding to the analyte such that the binding of the labelled reagent to the first reagent via the analyte is directly proportional to the concentration of the analyte, and
   the second chamber containing a second reagent proximal to the transducer, wherein the second reagent mimics the analyte such that the binding of the labelled reagent to the second reagent is inversely proportional to the concentration of the analyte;
(iii) exposing the sample to the transducer;
(iv) irradiating the sample with electromagnetic radiation and detecting the electrical signal, wherein the first and second reagents act as dynamic controls for each other.

Thus, the present invention provides a method for performing a sandwich assay in which the dynamic range and/or sensitivity of the assay can be extended by running a competitive assay simultaneously and combining the results.

WO2012/137009 describes a method for improving the accuracy and precision of an assay using a pyroelectric transducer by incorporating control measurements.

WO 2007/107716 relates to a method for detecting an analyte in a sample of whole blood using a pyroelectric transducer

US2012/220023 describes a method for making electrical contacts to a piezoelectric transducer in order to minimise vibrational noise generation.

The present invention will now be described with reference to the drawings, in which:
Fig. 1 shows a schematic representation of the chemical sensing device of WO 2004/090512 which is used with the present invention;
Fig. 2 shows a sandwich immunoassay using the device of the present invention;
Fig. 3 shows a cartridge according to the present invention;
Fig. 4 shows individual spot 2 counts without using controls;
Fig. 5 shows individual spot 3 counts; and
Fig. 6 shows dose-response curves using both positive and negative controls.

The method of the present invention is used for detecting an analyte in a sample. The method provides a device having: a radiation source adapted to generate a series of pulses of electromagnetic radiation; a transducer having a pyroelectric or piezoelectric element and electrodes, which is capable of transducing energy generated by non-radiative decay into an electrical signal; and a detector which is capable of detecting the electrical signal generated by the transducer. The device used in Z the present invention is based on the device described in WO 2004/090512.

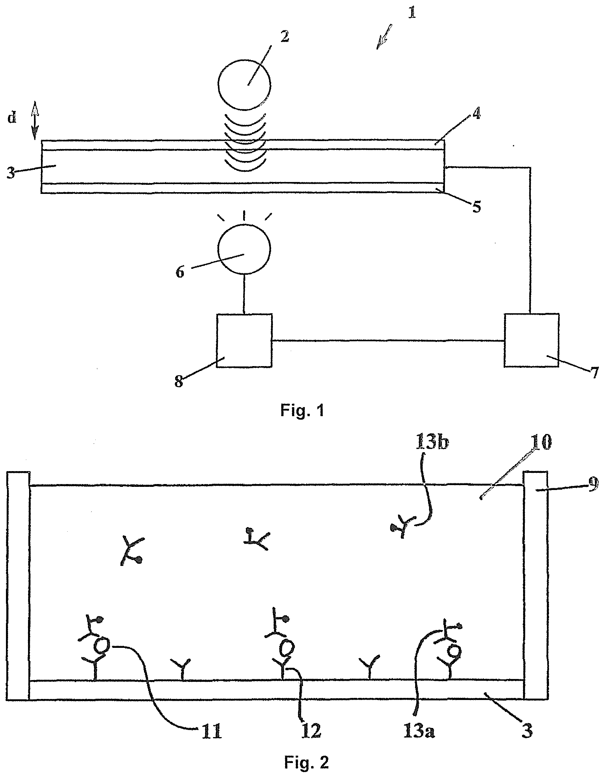

Fig. 1 shows a chemical sensing device 1 for use in accordance with the present invention which relies on heat generation in a label 2 on irradiation of the label 2 with electromagnetic radiation. For the sake of simplicity, only the label is shown in Fig. 1 (the remaining components of the device of the present invention will be described in further detail hereinbelow). Fig. 1 shows the chemical sensing device 1 in the presence of a label 2. The device 1 comprises a pyroelectric or piezoelectric transducer 3 having electrode coatings 4,5. The transducer 3 is preferably a poled polyvinylidene fluoride film. The electrode coatings 4,5 are preferably transparent and most preferably formed from indium tin oxide. The electrodes preferably have a thickness of about 20-30 nm, although almost any thickness is possible from a lower limit of 1 nm below which the electrical conductivity is too low and an upper limit of 200 nm above which the optical transmission is too low (it should not be less than 80%T). Preferably the transducer is an indium tin oxide-coated polyvinylidene fluoride film.

The label 2 is held proximal to the transducer 3 by a binding event. A preferred feature of the present invention is that the label 2 generates heat when irradiated by a source of electromagnetic radiation (typically termed "light") 6, preferably visible light. The light source may be, for example, an LED. The light source 6 illuminates the label 2 with light of the appropriate wavelength (e.g. a complementary colour). Although not wishing to be bound by theory, it is believed that the label 2 absorbs the light to generate an excited state which then undergoes non-radiative decay thereby generating energy, indicated by the curved lines in Fig. 1. This energy is primarily in the form of heat (i.e. thermal motion in the environment) although other forms of energy, e.g. a shock wave, may also be generated. The energy is, however, detected by the transducer and converted into an electrical signal. The device of the present invention is calibrated for the particular label being measured and hence the precise form of the energy generated by the non-radiative decay does not need to be determined. Unless otherwise specified the term "heat" is used herein to mean the energy generated by non-radiative decay. The light source 6 is positioned so as to illuminate the label 2. Preferably, the light source 6 is positioned opposite the transducer 3 and electrodes 4,5 and the label 2 is illuminated through the transducer 3 and electrodes 4,5. The light source may be an internal light source within the transducer in which the light source is a guided wave system. The wave guide may be the transducer itself or the wave guide may be an additional layer attached to the transducer. The wavelength of illumination depends on the label used; for example, for 40 nm gold labels the preferred wavelength is 525 nm and for carbon labels the preferred wavelength is 690 nm.

The energy generated by the label 2 is detected by the transducer 3 and converted into an electrical signal. The electrical signal is detected by a detector 7. The light source 6 and the detector 7 are both under the control of the controller 8. The light source 6 generates a series of pulses of light (the term "light" used herein means any form of electromagnetic radiation unless a specific wavelength is mentioned) which is termed "chopped light". In principle, a single flash of light, i.e. one pulse of electromagnetic radiation, would suffice to generate a signal from the transducer 3. However, in order to obtain a reproducible signal, a plurality of flashes of light are used which in practice requires chopped light. The frequency at which the pulses of electromagnetic radiation are applied may be varied. At the lower limit, the time delay between the pulses must be sufficient for the time delay between each pulse and the generation of an electrical signal to be determined. At the upper limit, the time delay between each pulse must not be so large that the period taken to record the data becomes unreasonably extended. Preferably, the frequency of the pulses is from 1-50 Hz, more preferably 1-10 Hz and most preferably 2 Hz. This corresponds to a time delay between pulses of 20-1,000 ms, 100-1,000 ms and 500 ms, respectively. In addition, the so-called "mark-space" ratio, i.e. the ratio of on signal to off signal is preferably one although other ratios may be used without deleterious effect. There are some benefits to using a shorter on pulse with a longer off signal, in order to allow the system to approach thermal equilibrium before the next pulse perturbs the system. In one embodiment, a light pulse of 1-50 ms, preferably 8 ms, followed by a relaxation time of 10-500 ms, preferably 250 ms allows a more precise measurement of particles bound directly to the surface. Sources of electromagnetic radiation which produce chopped light with different frequencies of chopping or different mark-space ratios are known in the art. The detector 7 determines the time delay between each pulse of light from light source 6 and the corresponding electrical signal detected by detector 7 from transducer 3. The applicant has found that this time delay is a function of the distance, d. The signal is preferably measured from 2-7 ms.

Any method for determining the time delay between each pulse of light and the corresponding electrical signal which provides reproducible results may be used. Preferably, the time delay is measured from the start of each pulse of light to the point at which a maximum in the electrical signal corresponding to the absorption of heat from bound label is detected as by detector 7.

The label 2 may be separated from the transducer surface and a signal may still be detected. The signal was detectable through an intervening medium capable of transmitting energy to the transducer 3, and different distances, d, may be distinguished (this has been termed "depth profiling") and that the intensity of the signal received is proportional to the concentration of the label 2 at the particular distance, d, from the surface of the transducer 3. Moreover, it was found that the nature of the medium itself influences the time delay and the magnitude of the signal at a given time delay. In the present invention, the detector is arranged to detect only the electrical signal corresponding to non-radiative decay occurring proximal to the transducer.

By way of an explanation of the principle underlying the present invention, Fig. 2 shows a typical capture antibody assay using the device of the present invention (although only the first reagent is shown). The device includes a transducer 3, a sample chamber 9 for holding a sample 10 containing an analyte 11 present therein and a capture reagent 12 proximal to the transducer surface. The transducer has a capture reagent 12, in the figure, an antibody, attached thereto. This attachment of the capture reagent 12 may be via a covalent bond or non-covalent adsorption onto the surface, such as by hydrogen bonding. An additional layer may separate the capture reagent 12 and the transducer 3, such as a parylene polymer layer, or the antibody could be attached to inert particles and the inert particles are then attached to the transducer 3. Alternatively, the capture reagent 12 could be entrapped within a gel layer which is coated onto the surface of the transducer 3.

In use, the sample chamber is filled with liquid 10 (or any fluid) containing an analyte 11. The analyte 11 then binds to first reagent 12. Additional labelled reagent 13 is present in the liquid and a so-called "sandwich" complex is formed between the bound first reagent 12, the analyte 11 and the labelled reagent 13. An excess of labelled reagent 13 is included so that all of the bound antigen 11 forms a sandwich complex. The sample therefore contains bound labelled reagent 13a and unbound labelled reagent 13b free in solution.

During or following formation of the sandwich complex, the sample is irradiated using a series of pulses of electromagnetic radiation, such as light. The time delay between each pulse and the generation of an electrical signal by the transducer 3 is detected by a detector. The appropriate time delay is selected to measure primarily the heat generated by the bound labelled reagent 13a. Since the time delay is a function of the distance of the label from the transducer 3, the bound labelled reagent 13a may be distinguished from the unbound labelled reagent 13b. This provides a significant advantage over the conventional sandwich immunoassay in that it removes the need for washing steps. In a conventional sandwich immunoassay, the unbound labelled reagent must be separated from the bound labelled reagent before any measurement is taken since the unbound labelled reagent interferes with the signal generated by the bound labelled reagent. However, on account of the "depth profiling" provided by the present invention, bound and unbound labelled reagent may be distinguished. Indeed, the ability to distinguish between labels proximal to the transducer (i.e. bound) and labels in the bulk solution (i.e. unbound) is a particular advantage of the present invention.

The method of the present invention also comprises exposing the sample to the device as described herein, transducing the energy generated into an electrical signal and detecting the signal. Preferably, the method is carried out without removing the sample from the transducer between the steps of exposing the sample to the transducer and transducing the energy generated into an electrical signal, i.e. the method is a homogeneous assay.

In a preferred embodiment, the device used in the present invention is based on the device described in WO 2012/137009.

The device used in Z the present invention comprises a first, second and third chamber, which contain a first, second and third reagent, respectively, proximal to the transducer, wherein the first and second reagents act as dynamic controls for each other.

The first reagent has a binding site which is capable of binding to the analyte. The labelled reagent then binds to the analyte such that the binding of the labelled reagent to the first reagent via the analyte is directly proportional to the concentration of the analyte. In this manner, the analyte has two differing epitopes. Accordingly, the first chamber comprises a sandwich immunoassay.

Determining the extent of binding of the labelled reagent to the first reagent provides a measurement of the concentration of the analyte in the sample.

The second reagent mimics the analyte and is an analogue of the analyte (it is an analogue because it is bound to the transducer surface either through covalent bonding or non-covalent interactions). The labelled reagent binds directly to the second reagent. The second reagent will compete with any free analyte in the sample for the binding of the labelled reagent such that the binding of the labelled reagent to the second reagent is inversely proportional to the concentration of the analyte in the sample. When no analyte is present in the sample, the labelled reagent binds exclusively to the second reagent and a high signal is detected. With increasing concentration of analyte present in the sample, binding of the labelled reagent to the second reagent proportionally decreases and a drop in signal is detected. Accordingly, the second chamber comprises a competitive immunoassay. The present invention is therefore a combination assay as both a sandwich and competitive immunoassay are used to detect an analyte in a sample, wherein the first and second reagent act as dynamic controls for each other.

The sensitivity of the competitive assay as a function of analyte concentration depends upon a number of factors, including the population of labelled reagent and the surface concentration of the second reagent. By adjusting these factors it is possible to "tune" the sensitivity of the competitive assay.

In one embodiment, the competitive assay is tuned so that it is less sensitive to the analyte concentration than the sandwich assay. In this manner, as the concentration of the analyte present in the sample increases, the signal from the first sample chamber rises sharply whereas the signal from the second chamber falls slowly. By combining these two outputs mathematically, it is possible to maintain the detection limit of the sandwich assay, whilst extending the dynamic range of the assay by use of the less sensitive competition assay that is taking place simultaneously.

In a second embodiment, the sandwich assay and the competitive assay have similar sensitivities, and hence they saturate at similar concentrations. In this instance, the dynamic range of the assay is not enhanced, but the detection limit is improved by combining two assays that are functioning in opposite modes.

Preferably, the device further comprises a third chamber comprising a third reagent proximal to the transducer, the third reagent having a lower affinity for the analyte or the labelled reagent under the conditions of the assay than the first reagent for the analyte. Accordingly, the third reagent provides the negative control. It is important that the affinity is considered under the conditions of the assay. The reason is that the affinity of the first reagent for the labelled reagent is mediated by the presence of the analyte. Thus, in the absence of the analyte, neither the first nor third reagent has any affinity for the labelled reagent. However, in the presence of the analyte, the third reagent has a lower affinity for the labelled reagent than the first reagent.

The third reagent is an inert surface which mimics the surface where the first and second reagents are immobilised. In one embodiment, the third reagent is a polysaccharide, for example dextran. In an alternative embodiment, the third reagent is an isotype control antibody. The third reagent typically has similar chemical and physical properties to the first reagent, but provides little or no affinity for the labelled reagent under the conditions of the assay. In a particularly preferred embodiment, the third reagent has essentially no affinity for the labelled reagent under the conditions of the assay. Preferably, the third reagent provides essentially no affinity for the analyte. That is, the binding of the labelled reagent, or, where applicable, the analyte, to the third reagent is non-specific. In this manner, the third reagent can compensate for non-specific binding of the labelled reagent to the first reagent, and can also compensate for unwanted movement of the labelled reagent relative to the transducer, e.g. by sedimentation under gravity, which can interfere with the measurement process.

The electrical signals from the sample chambers are manipulated mathematically. The mathematical manipulation of the electrical signals is the same as that described in WO 2012/137009. In a preferred embodiment, the method of the present invention further comprises subtracting the electrical signal of the third chamber from the electrical signals of the first and second chambers to obtain baseline corrected electrical signals and dividing the baseline corrected electrical signal of the first chamber by the baseline corrected electrical signal of the second chamber.

In the present invention, the first and second reagents can be considered as acting as "dynamic" controls for each other, whereas WO 2012/137009 uses a "static" positive control. When using such a static positive control, the algorithmic output is limited approximately to lie between 0.000 and 1.000.

In the present invention, at low concentrations of analyte, a ratiometric signal between 0.000 and 1.000 is obtained which defines the magnitude of the signal from binding to the first reagent (i.e. the measurement signal) relative to the binding of the second and third reagents (i.e. the positive and negative controls, respectively), by interrogating the output of the detector.

In one embodiment, at high concentrations of analyte, the ratiometric signal increases above 1.000 i.e. the signal from binding to the first reagent exceeds the signal from binding to the second reagent. This arises because the signal from binding to the second reagent (the positive control) decreases slowly whilst the signal from binding to the first reagent (the measurement signal) increases sharply. In this embodiment, the decrease in the signal from binding to the second reagent is steady owing to the use of a low sensitivity competitive assay. The ratiometric signal continues to increase above 1.000 until the signal from binding to the second reagent approaches 0.000 owing to saturation of the labelled reagent with analyte. In this manner, the first and second reagents are working in combination with each other to extend the dynamic range of the sandwich assay in the first sample chamber and delay the onset of saturation and/or high dose hook which would normally be observed at lower analyte concentrations. The first and second reagents can be thought of as acting as dynamic controls for each other. In combination with the third reagent, the first and second reagents are acting to increase the dynamic range of a sandwich assay without compromising the sensitivity of the assay.

In a preferred embodiment, the binding of the first and second reagents to the analyte or the labelled reagent is dependent on the analyte concentration and the binding of the third reagent to the analyte or the labelled reagent is independent of analyte concentration. The second reagent (the positive control) is therefore variable whereas the third reagent (the negative control) is constant. The present invention removes the requirement for a "static" positive control, although such a control may also be included without detriment.

The first, second and third reagents may be attached to the transducer using techniques known in the art. Preferably the attachment is via non-covalent bonding, for example, a primary layer such as streptavidin or polystreptavidin, is adsorbed on to the transducer and the reagents are attached to the primary layer by a binding event.

The assay also requires the presence of a labelled reagent. By "labelled" reagent is meant a reagent which is attached to a label, which label being capable of absorbing the electromagnetic radiation generated by the radiation source to generate energy by non-radiative decay. It is this non-radiative decay which is transduced into an electrical signal by the transducer.

The label may therefore be composed of any material which is capable of interacting with the electromagnetic radiation in this manner. Preferably the label is selected from, but not limited to, a carbon particle, a coloured-polymer particle (e.g. coloured latex), a dye molecule, an enzyme, a fluorescent molecule, a metal (e.g. gold) particle, a haemoglobin molecule, a red blood cell, a magnetic particle, a nanoparticle having a non-conducting core material and at least one metal shell layer, a particle composed of polypyrrole or a derivative thereof, and combinations thereof. Preferably, the label is a carbon particle or a gold particle and most preferably a carbon particle.

In the case of a magnetic particle, the electromagnetic radiation is radio frequency radiation. All of the other labels mentioned hereinabove employ light, which can include IR or UV radiation. Gold particles are commercially available or may be prepared using known methods (see for example G. Frens, Nature, 241, 20-22 (1973)). For a more detailed explanation of the nanoparticle label see US 6,344,272 and WO 2007/141581.

Preferably, the present invention uses a particle having a particle size of 20 to 1,000 nm, more preferably 100 to 500 nm. By particle size is meant the diameter of the particle at its widest point. The density of the particle will depend on the type of assay. Where the assay is diffusion-controlled, the particle preferably has a density of 0.5 to 3.0 g/mL, more preferably 1.5-2.0 g/mL and most preferably 1.8 g/mL. In this assay type, the particle is a carbon particle having the aforementioned particle size and density. Where the assay is gravity-assisted, the particle preferably has a density of 1.5 to 23 g/mL, more preferably 15-20 g/mL and most preferably 19 g/mL. In this assay type, the particle is a gold particle having the aforementioned particle size and density.

The label is proximal to the transducer when the binding event has occurred. That is, the label is sufficiently close to the surface of the transducer for the transducer to be able to detect the energy generated by the label on irradiation of the sample. The actual distance between the label and the surface of the transducer will, however, depend on a number of variables, such as the size and nature of the label, the size and nature of the antibodies and the analyte, the nature of the sample medium, and the nature of the electromagnetic radiation and the corresponding settings of the detector. The device of the present invention may include a radiation source which is adapted to generate a series of pulses of electromagnetic radiation and the detector is adapted to determine the time delay between each pulse of electromagnetic radiation from the radiation source and the generation of the electric signal thereby allowing a precise determination of the position of the label with respect to the transducer as discussed with reference to Fig. 1.

The nature of the first, second and third reagents, as well as the labelled reagent, will depend on the nature of the analyte. In a particularly preferred embodiment, the labelled reagent comprises an antibody raised to the analyte or the complex or derivative of the analyte, the first reagent is an antibody raised to the analyte or the complex or derivative of the analyte, the second reagent is an analogue of the analyte, and the third reagent is a neutral surface. In principle, a single molecule could be used for each reagent, but in practice, the first, second and third reagents, as well as the labelled reagent, are a population of molecules. The term "antibody" preferably includes within its scope a Fab fragment, a single-chain variable fragment (scFv), and a recombinant binding fragment.

As alternatives to antibody-antigen reactions, the reagents and analyte may be a first and second nucleic acid where the first and second nucleic acids are complementary, or a reagent containing avidin or derivatives thereof and an analyte containing biotin or derivatives thereof, or vice versa. The reagents may also be aptamers. The system is also not limited to biological assays and may be applied, for example, to the detection of heavy metals in water. The system also need not be limited to liquids and any fluid system may be used, e.g. the detection of enzymes, cells and viruses etc. in the air.

The maximum observable signal is the maximum signal that can be achieved when monitoring the label binding to a surface. In the absence of alternative mass transport phenomena (e.g. convection, magnetic movement, buoyancy, sedimentation, etc.), the binding of particles to the transducer is governed by the diffusion rate of the analyte and labelled reagent which is, in turn, governed largely by the hydrodynamic radius of these components and the viscosity/temperature of the sample. The negative control should give a signal that is independent of the absence or presence of the analyte to be measured.

It has been found that for immunometric (i.e. sandwich or reagent-excess) assays, improvements in performance can be achieved by using an analogue of an analyte as a dynamic positive control (that recognises an antibody on the labelled reagent), and a nonreactive surface as the negative control.

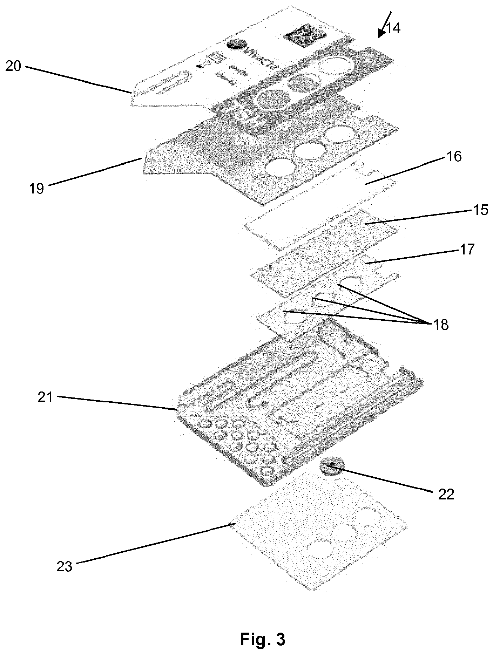

The sample which is suspected of containing the analyte of interest will generally be a fluid sample, e.g. a liquid sample, and usually a biological sample, such as a bodily fluid, e.g. blood, plasma, saliva, serum or urine. The sample may contain suspended particles and may even be whole blood. An advantage of the method of the present invention is that the assay may be performed on a sample which does contain suspended particles without unduly influencing the results of the assay. The sample will typically be in the order of microlitres (e.g. 1-100 µL, preferably 1-10 µL). In order to hold a fluid sample, the transducer is preferably located in a sample chamber having one or more side walls, an upper surface and a lower surface. Accordingly, the device of the present invention preferably further comprises a chamber for holding a liquid sample containing the analyte or the complex or derivative of the analyte in contact with the transducer. In a preferred embodiment, the transducer is integral with the chamber, i.e. it forms one of the side walls, or upper or lower surface which define the chamber. Preferably the transducer forms the upper surface as shown in Fig. 3. Clearly, the first, second and third reagents and the labelled reagent will be on the interior surfaces of the chamber to allow contact with the sample. The sample may simply be retained by surface tension forces, for example, inside a capillary channel.

The device contains a first chamber containing the first reagent, a second chamber containing the second reagent and a third chamber containing the third reagent. The first, second and third reagents are proximal to the transducer. The first, second and third chambers are preferably in fluid communication, allowing the mixture of sample and labelled reagents to fill the three chambers in a sequential fashion. It should be noted that the sample and labelled reagents in each of the three chambers are identical in the present invention. The device preferably further contains a capillary channel having a sample receiving end which is contact with the outside of the device and a sample delivery end which is in fluid communication with the sample chamber(s), as shown in the core 21 in Fig. 3.

The labelled reagent and optionally one or more additional reagents are preferably stored in a chamber incorporated into the device of the present invention. The labelled reagent may also be supplied as part of a kit incorporating the device and the labelled reagent. Accordingly, the present invention also provides a kit comprising the device as described herein and the labelled reagent. The labelled reagent may be deposited onto the surface of the transducer.

The device used in the present invention is not restricted to detecting only one analyte and different analytes may be detected by employing different first reagents which selectively bind each analyte, or a derivative or complex of the analyte, being detected. Multiple tests can be carried out using only one electrical connection to the transducer, by illuminating different locations of the transducer sequentially and interrogating the outputs sequentially.

A potential additional source of background interference is the settling of suspended particles on to the surface of the piezo/pyroelectric transducer, including labelled reagent and cellular components of the sample. This source of interference may be reduced by positioning the transducer above the bulk solution, e.g. on the upper surface of the reaction chamber. Thus, if any settling occurs, it will not interfere with the transducer. Alternatively, the particles could be less dense than the medium and hence float to the surface of the bulk solution rather than settling on the surface of the transducer. This and other modifications are included in the scope of the present invention.

The device used in the present invention consists essentially of the above-described features. By "essentially" is meant that no other features are required to perform the assay. The device may take the form of a separate reader and cartridge, or an integrated device. In the former, the device is formed of a reader and a cartridge, in which the cartridge is releasably engageable with the reader, and in which the reader incorporates the radiation source and the detector, and the cartridge incorporates the transducer and the first, second and third reagents. The reader is preferably a portable reader. The present invention also provides the cartridge comprising the transducer and the first, second and third reagents as defined herein. The cartridge is preferably a disposable cartridge.

The present invention will now be described with reference to the following examples which are not intended to be limiting.

### Examples

### PVDF film

A poled piezo/pyroelectric polyvinylidene fluoride (PVDF) bimorph film, coated in indium tin oxide was used as the sensing device in the following examples. The indium tin oxide surface was coated with a layer of parylene (of approximate thickness 1 micron) by a vapour phase gas deposition process. This method involved the sublimation and subsequent pyrolysis of a paracyclophane precursor, followed by a free-radical polymerisation on the surface. See WO 2009/141637 for further details. The resulting parylene layer was then coated with a layer of biotinylated bovine serum albumin (10 µg/mL in 10 mM phosphate buffer) by passive adsorption over the course of two hours. The surface was washed and then coated in polystreptavidin solution (10 µglmL in PBS-10 mmol/L phosphate buffer containing 2.7 mmol/L KCI, 137 mmol/L NaCl and 0.05% Tween) by incubation at room temperature overnight. Polystreptavidin was prepared as described by Tischer et al (U.S. Pat. No. 5,061,640).

### Materials

Monoclonal antibodies were raised essentially as described in "Monoclonal Antibodies: Properties, Manufacture and Applications" by J. R. Birch and E. S. Lennox, Wiley-Blackwell, 1995, and biotinylated by methods known in the art. Carbon-labelled reporter conjugates were prepared essentially as described by Van Doom et al. (U.S. Pat. No. 5,641,689).

### Preparation of the cartridge

Strips of PVDF pyroelectric polymer film were coated in three separate areas with a universal streptavidin coating, as described above. The three areas were separated by an adhesive spacer attached to the surface of the sensor, allowing subsequent incubation of reagents onto each area without cross-contamination of the surfaces. The three surfaces (labelled spot 1, spot 2 and spot 3) were coated with three different biotinylated reagents, washed and then dried in the presence of sucrose stabiliser.

As shown in FIG. 3, a cartridge 14 was fabricated to perform the assay. The cartridge 14 was fabricated from an antibody-coated piezo/pyrofilm 15 supported on a stiffener 16. A pressure sensitive adhesive-coated polyester film 17 die-cut to form three sample chambers 18 was applied to the surface. Provision was made to allow for electrical connections to the top and bottom surfaces of the piezo/pyrofilm 15 in order to detect the charge generated. The cartridge 14 was then formed by sandwiching the above components between a top cover 19, to which a label 20 was applied, and a core 21, seal 22 and bottom cover 23.

### Assays

Assays were carried out by charging the sample chambers with the sample through the capillary channel in the core 21. The sample was either pre-mixed with the labelled carbon particles prior to being loaded into the cartridge, or the carbon particles (and any additional reagents) were dried down in the channels of the cartridge. The sample was then mixed with the reagents by being pumped through the channels of the cartridge by a displacement pump in the instrument. After the measurement chambers had filled, they were then irradiated (through the holes in the top cover 19) sequentially with chopped LED light. For each LED pulse, a voltage was measured across the piezo/pyrofilm 15 using an amplifier and analogue to digital (ADC) converter. The change in the ADC signal is calculated over time. The final output from each spot is the rate of change of signal from the ADC as a function of time.

### Example 1 (comparative) and Example 2 (invention)

Two different types of assay for IgE were carried out. Each assay used three measurement chambers, denoted spot 1 (negative control), spot 2 (sandwich assay) and spot 3 (positive control). The coatings in spots 1 and 2 were identical for each assay. Spot 1 was coated with a biotin conjugated 70 kDa amino-dextran (Fleet Bioprocessing) at a concentration of 1 µg/ml and spot 2 was coated with a biotinylated anti-lgE Hytest mouse antibody (clone 4F4) at a concentration of 4 µg/ml. The coatings in spot 3 were specific to each assay. In Example 1 (comparative), with a fixed positive control, spot 3 was coated with biotinylated goat anti-mouse IgG. In Example 2 (invention), biotinylated IgE was coated onto spot 3 at a concentration of 10 µg/ml. Carbon particles coated in a matching anti-lgE antibody, Hytest 4F4, were used as the label in the system. For the avoidance of confusion, the surface of spot 2 (described as the first chamber above) is equivalent to the first reagent, the surface of spot 3 (described as the second chamber above) is equivalent to the second reagent and the surface of spot 1 (described as the third chamber above) is equivalent to the third reagent.

Repeat measurements (six at each concentration) were carried out on fresh blood samples at four different IgE concentrations (0, 156, 547, 1471 IU/mL). For each cartridge, the output from spot 1 was subtracted from the outputs in both spot 2 and spot 3 (i.e. both measurements were baseline corrected). Then the baseline corrected measurement in spot 2 was divided by the baseline corrected measurement in spot 3. The mean final assay count (and standard deviation) was then calculated from the cartridges run at each concentration.

The results are set out in Table 1.

**Table 1. Mean final assay counts and individual spot outputs for two IgE assays**

| | **IgE Concentration (IU/mL)** | **Mean Final Assay Counts** | **Spot 1 (mean)** | **Spot 2 (mean)** | **Spot 3 (mean)** |
|---|---|---|---|---|---|
| **Example 1 (Comparative) Goat Anti-Mouse Spot 3** | 0 | 0.0431 | -419.3905 | 50.5571 | 10702.0851 |
| | 156 | 0.5224 | -233.5235 | 5941.9443 | 11590.9966 |
| | 547 | 0.6859 | -415.1160 | 7954.2608 | 11800.8352 |
| | 1471 | 0.5963 | -280.7532 | 6256.8410 | 10686.1634 |
| **Example 2 (Invention) Biotinylated IgE Spot 3** | 0 | 0.0438 | -115.5008 | 481.3997 | 13517.8891 |
| | 156 | 0.8225 | -309.9573 | 8071.0033 | 9894.9731 |
| | 547 | 1.6244 | -283.0646 | 11221.4316 | 6799.9965 |
| | 1471 | 3.8582 | -210.2222 | 9487.1137 | 2302.3897 |

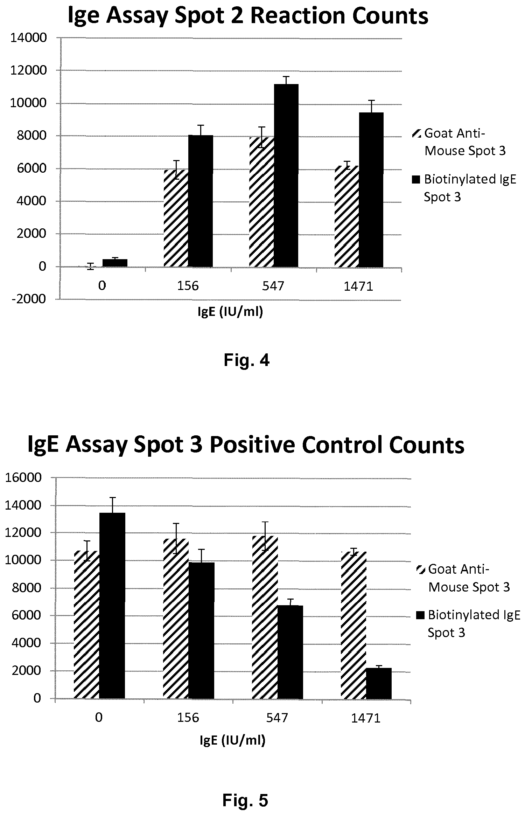

Figure 4 shows that the individual spot 2 counts of Example 1 (comparative), without using the dynamic controls of the present invention, give rise to a saturation and slight hooking at IgE concentrations between 547 and 1471 IU/mL.

Figure 5 compares the individual spot 3 counts for Example 1 (comparative) and Example 2 (invention). The results show that the spot 3 count for Example 1 (comparative) remains high, independent of analyte concentration, and that the spot 3 count for Example 2 (invention) decreases with increasing analyte concentration.

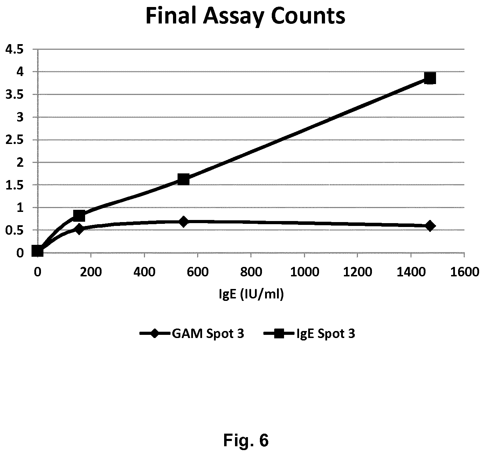

Figure 6 shows the dose-response curve for the data using both controls. Example 1 (comparative) shows a much narrower dynamic range (saturating around 200 IU/mL) whereas Example 2 (invention) shows a much wider dynamic range, measuring up to 1500 IU/mL.

## Claims

1. A method for detecting an analyte in a sample using electromagnetic radiation comprising:
(i) providing a labelled reagent, the labelled reagent having a binding site which is capable of binding the analyte or an analogue of the analyte and a label, wherein the label is capable of absorbing the electromagnetic radiation to generate energy by non-radiative decay;
(ii) providing a device having
a radiation source adapted to generate a series of pulses of electromagnetic radiation,
a transducer having a pyroelectric or piezoelectric element and electrodes, which is capable of transducing energy generated by non-radiative decay into an electrical signal,
a detector which is capable of detecting the electrical signal,
a controller for controlling the source of electromagnetic radiation and the detector,
wherein the device has a first and second chamber,
the first chamber containing a first reagent proximal to the transducer, wherein the first reagent is capable of binding to the analyte such that the binding of the labelled reagent to the first reagent via the analyte is directly proportional to the concentration of the analyte, and the second chamber containing a second reagent proximal to the transducer, wherein the second reagent mimics the analyte such that the binding of the labelled reagent to the second reagent is inversely proportional to the concentration of the analyte;
(iii) exposing the sample to the transducer;
(iv) irradiating the sample with electromagnetic radiation and detecting the electrical signal, wherein the first and second reagents act as dynamic controls for each other.

2. A method as claimed in claim 1, wherein the device further comprises a third chamber comprising a third reagent proximal to the transducer, the third reagent having a lower affinity for the analyte or the labelled reagent under the conditions of the assay than the first reagent for the analyte.

3. A method as claimed in claim 1 or 2, wherein the binding of the first and second reagents to the analyte or the labelled reagent is dependent on the analyte concentration and the binding of the third reagent to the analyte or the labelled reagent is independent of analyte concentration.

4. A method as claimed in any preceding claim, wherein the third reagent has essentially no affinity for the analyte or the labelled reagent under the conditions of the assay.

5. A method as claimed in any preceding claim, wherein the first, second and third reagents are attached to the transducer via non-covalent bonding.

6. A method as claimed in any preceding claim, wherein the first reagent is an antibody and the analyte is an antigen.

7. A method as claimed in any preceding claim, wherein the labelled reagent is a labelled antibody.

8. A method as claimed in any preceding claim, wherein the labelled reagent comprises a label, wherein the label is selected from a dye molecule, a gold particle, a coloured-polymer particle, a fluorescent molecule, an enzyme, a red blood cell, a haemoglobin molecule, a magnetic particle and a carbon particle.

9. A method as claimed in any preceding claim, wherein the method is carried out without removing the sample from the transducer between the steps of exposing the sample to the transducer and irradiating the sample.

10. Use in a method of claims 1-9 of a kit comprising the device and the labelled reagent, as claimed in any preceding claim.

## Patentansprüche

1. Verfahren zum Nachweisen eines Analyten in einer Probe mittels elektromagnetischer Strahlung, umfassend:
(i) ein Bereitstellen eines markierten Reagenz, wobei das markierte Reagenz eine Bindungsstelle, die in der Lage ist, den Analyten oder ein Analogon des Analyten zu binden, und eine Markierung aufweist, wobei die Markierung in der Lage ist, die elektromagnetische Strahlung zu absorbieren, um durch nicht-strahlenden Zerfall Energie zu erzeugen;
(ii) ein Bereitstellen einer Vorrichtung mit einer Strahlungsquelle, die angepasst ist, um eine Reihe von Impulsen elektromagnetischer Strahlung zu erzeugen, einen Wandler mit einem pyroelektrischen oder piezoelektrischen Element und Elektroden, der in der Lage ist, durch nicht-strahlenden Zerfall erzeugte Energie in ein elektrisches Signal zu wandeln, einen Detektor, der in der Lage ist, das elektrische Signal nachzuweisen, eine Steuerung zum Steuern der Quelle elektromagnetischer Strahlung und des Detektors, wobei die Vorrichtung eine erste und eine zweite Kammer aufweist, wobei die erste Kammer einen ersten Reagenz proximal zum Wandler enthält, wobei der erste Reagenz in der Lage ist, sich an den Analyten zu binden, so dass die Bindung des markierten Reagenz zum ersten Reagenz über den Analyten direkt proportional zur Konzentration des Analysten ist, und die zweite Kammer einen zweiten Reagenz proximal zum Wandler enthält, wobei der zweite Reagenz den Analysten nachahmt, so dass die Bindung des markierten Reagenz an den zweiten Reagenz umgekehrt proportional zur Konzentration des Analysten ist;
(iii) ein Exponieren der Probe gegenüber dem Wandler;
(iv) ein Bestrahlen der Probe mit elektromagnetischer Strahlung und ein Nachweisen des elektrischen Signals, wobei der erste und der zweite Reagenz als dynamische Steuerungen füreinander fungieren.

2. Verfahren nach Anspruch 1, wobei die Vorrichtung ferner eine dritte Kammer umfasst, die einen dritten Reagenz proximal zum Wandler enthält, wobei der dritte Reagenz eine niedrigere Affinität zum Wandler oder zum markierten Reagenz unter den Versuchsbedingungen aufweist als der erste Reagenz zum Analyten.

3. Verfahren nach Anspruch 1 oder 2, wobei die Bindung des ersten und zweiten Reagenz an den Analyten oder den markierten Reagenz von der Analytenkonzentration abhängig ist und die Bindung des dritten Reagenz an den Analyten oder den markierten Reagenz von der Analytenkonzentration unabhängig ist.

4. Verfahren nach einem der vorangehenden Ansprüche, wobei der dritte Reagenz im Wesentlichen keine Affinität zum Analyten oder zum markierten Reagenz unter den Versuchsbedingungen aufweist.

5. Verfahren nach einem der vorangehenden Ansprüche, wobei der erste, zweite und dritte Reagenz über eine nicht-kovalente Bindung am Wandler angebracht ist.

6. Verfahren nach einem der vorangehenden Ansprüche, wobei der erste Reagenz ein Antikörper ist und der Analyt ein Antigen ist.

7. Verfahren nach einem der vorangehenden Ansprüche, wobei der markierte Reagenz ein markierter Antikörper ist.

8. Verfahren nach einem der vorangehenden Ansprüche, wobei der markierte Reagenz eine Markierung umfasst, wobei die Markierung ausgewählt ist aus einem Farbstoffmolekül, einem Goldpartikel, einem gefärbten Polymerpartikel, einem fluoreszenten Molekül, einem Enzym, einer roten Blutzelle, einem Hämoglobinmolekül, einem magnetischen Partikel und einem Kohlenstoffpartikel.

9. Verfahren nach einem der vorangehenden Ansprüche, wobei das Verfahren ohne Entfernen der Probe aus dem Wandler zwischen den Schritten des Exponierens der Probe gegenüber dem Wandler und des Bestrahlens der Probe ausgeführt wird.

10. Verwendung in einem Verfahren nach Ansprüche 1 bis 9 eines Kits, umfassend die Vorrichtung und den markierten Reagenz nach einem der vorangehenden Ansprüche.

## Revendications

1. Procédé permettant la détection d'une substance à analyser dans un échantillon à l'aide d'un rayonnement électromagnétique comprenant :
(i) la fourniture d'un réactif marqué, le réactif marqué possédant un site de liaison qui est capable de lier la substance à analyser ou un analogue de la substance à analyser et un marqueur, ledit marqueur étant capable d'absorber le rayonnement électromagnétique pour générer de l'énergie par décroissance non radiative ;
(ii) la fourniture d'un dispositif possédant une source de rayonnement adaptée pour générer une série d'impulsions de rayonnement électromagnétique, un transducteur possédant un élément pyroélectrique ou piézoélectrique et des électrodes, qui est capable de convertir l'énergie générée par la décroissance non radiative en un signal électrique, un détecteur qui est capable de détecter le signal électrique, un dispositif de commande destiné à commander la source de rayonnement électromagnétique et le détecteur, ledit dispositif possédant une première et une seconde chambre, la première chambre contenant un premier réactif à proximité du transducteur, ledit premier réactif étant capable de se lier à la substance à analyser de sorte que la liaison du réactif marqué au premier réactif par l'intermédiaire de la substance à analyser soit directement proportionnelle à la concentration de la substance à analyser, et la deuxième chambre contenant un deuxième réactif à proximité du transducteur, ledit deuxième réactif imitant la substance à analyser de sorte que la liaison du réactif marqué au deuxième réactif soit inversement proportionnelle à la concentration de la substance à analyser ;
(iii) l'exposition de l'échantillon au transducteur ;
(iv) l'irradiation de l'échantillon avec un rayonnement électromagnétique et la détection du signal électrique, lesdits premier et deuxièmes réactifs agissant comme des commandes dynamiques l'un pour l'autre.

2. Procédé selon la revendication 1, ledit dispositif comprenant en outre une troisième chambre comprenant un troisième réactif à proximité du transducteur, le troisième réactif possédant une affinité plus faible pour la substance à analyser ou le réactif marqué dans les conditions de l'essai que le premier réactif pour la substance à analyser.

3. Procédé selon la revendication 1 ou 2, ladite liaison des premier et deuxième réactifs à la substance à analyser ou au réactif marqué étant dépendante de la concentration de la substance à analyser et ladite liaison du troisième réactif à la substance à analyser ou au réactif marqué étant indépendante de la concentration de la substance à analyser.

4. Procédé selon l'une quelconque des revendications précédentes, ledit troisième réactif ne possédant essentiellement aucune affinité pour la substance à analyser ou le réactif marqué dans les conditions de l'essai.

5. Procédé selon l'une quelconque des revendications précédentes, lesdits premier, deuxième et troisième réactifs étant fixés au transducteur par l'intermédiaire d'une liaison non covalente.

6. Procédé selon l'une quelconque des revendications précédentes, ledit premier réactif étant un anticorps et ladite substance à analyser étant un antigène.

7. Procédé selon l'une quelconque des revendications précédentes, ledit réactif marqué étant un anticorps marqué.

8. Procédé selon l'une quelconque des revendications précédentes, ledit réactif marqué comprenant un marqueur, ledit marqueur étant choisi parmi une molécule de colorant, une particule d'or, une particule de polymère coloré, une molécule fluorescente, une enzyme, un globule rouge, une molécule d'hémoglobine, une particule magnétique et une particule de carbone.

9. Procédé selon l'une quelconque des revendications précédentes, ledit procédé étant effectué sans retirer l'échantillon du transducteur entre les étapes d'exposition de l'échantillon au transducteur et d'irradiation de l'échantillon.

10. Utilisation dans un procédé des revendications 1 à 9 d'un ensemble comprenant le dispositif et le réactif marqué, selon l'une quelconque des revendications précédentes.
